# EUROPÄISCHE PATENTSCHRIFT

(11) **EP 0 912 544 B1**
(45) Veröffentlichungstag und Bekanntmachung des Hinweises auf die Patenterteilung: **06.10.2004**
(21) Anmeldenummer: 96927534.6
(22) Anmeldetag: 09.07.1996
(51) Int. Cl.: C07D 319/06, C07D 319/08

(54) **VERFAHREN ZUR HERSTELLUNG VON 2-SUBSTITUIERTEN BUT-2-EN-1,4-DIAL-4-ACETALEN UND NEUE HALBACETALE VON GLYOXALMONOACETALEN**
PROCESS FOR PREPARING 2-SUBSTITUTED BUT-2-ENE-1,4-DIAL-ACETALS AND NOVEL HEMIACETALS OF GLYOXALMONOACETALS
PROCEDE DE PREPARATION DE BUT-2-ENE-1,4-DIAL-4-ACETALS 2-SUBSTITUES ET NOUVEAUX HEMI-ACETALS DE GLYOXALMONOACETALS

(43) Veröffentlichungstag der Anmeldung: 06.05.1999
(73) Patentinhaber: BASF AKTIENGESELLSCHAFT, 67056 Ludwigshafen (DE)
(72) Erfinder: FRANK, Jürgen, D-67067 Ludwigshafen (DE); MELDER, Johann-Peter, D-67459 Böhl-Iggelheim (DE); MERGER, Franz, D-67227 Frankenthal (DE); WITZEL, Tom, Ota-ku,Tokyo 145-0071 (JP)
(86) Internationale Anmeldenummer: PCT/EP1996/002989
(87) Internationale Veröffentlichungsnummer: WO 1998/001439

(56) Entgegenhaltungen:
- EP-A- 0 246 646
- EP-A- 0 249 530
- EP-A- 0 316 672
- "Ullmann's Encyclopedia of Industrial Chemistry, Volume A1," 1985 XP002025501 siehe Seite 336 - Seite 338

## Beschreibung

Die vorliegende Erfindung betrifft ein Verfahren zur Herstellung von But-2-en-1,4-dial-4-acetalen der allgemeinen Formel I 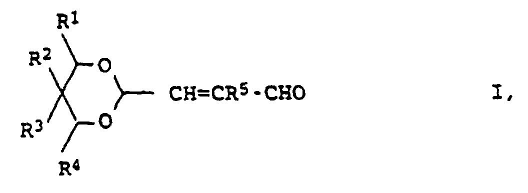 in der die Substituenten R¹ bis R⁴ für Wasserstoff oder C₁-C₆-aliphatische Reste stehen und R² und R³ oder R¹ und R² jeweils gemeinsame Glieder eines aliphatischen 4- bis 7-gliedrigen Ringes, der ein Heteroatom enthalten kann, sind, und R⁵ einen Alkyl-, Alkenyl- oder Alkinylrest mit 1 bis 12 C-Atomen, der durch cycloaliphatische, aromatische oder heterocyclische Reste oder durch Hydroxy-, Ether-, Thioether-, Acyl-, Alkylamino-, Carboxi- oder Carbalkoxygruppen substituiert sein kann, einen gegebenenfalls substituierten Arylrest oder eine Alkoxi-, Alkylthio- oder Acyloxygruppe bedeutet.

Weiterhin betrifft die Erfindung neue Halbacetale von Glyoxalmonoacetalen.

Die EP-A 249 530 lehrt die Herstellung von Glyoxalmonoacetalen aus Glyoxal und Alkoholen in Gegenwart von katalytischen Mengen Säure sowie eines molaren Überschusses Alkohol. Die EP-A 316 672 beschreibt die Herstellung der Glyoxalmonoacetale durch Umsetzung von Glyoxal mit 1,3-Propandiolderivaten in Gegenwart von Wasser und einer Säure bei Temperaturen bis 150°C. Nach der Lehre beider Schriften werden die Produkte vorwiegend destillativ isoliert. Dabei führt die Bildung hochsiedender Oligomere zu Ausbeuteverlusten, da diese nur zum Teil thermisch wieder monomerisieren. Zur Erzielung brauchbarer Gesamtausbeuten an Glyoxalmonoacetalen im technischen Maßstab ist es außerdem notwendig, alle destillativ isolierten Nebenprodukte und Ausgangsprodukte in die Reaktion zurückzuführen, denn nur so läßt sich das Gesamtverfahren wirtschaftlich gestalten.

Die EP-A 246 646 beschreibt die Herstellung von 4-Acetalen von But-2-en-1,4-dialen aus Glyoxalmonoacetalen und Aldehyden bei 20 bis 150°C. In einer bevorzugten Verfahrensweise wird diese Reaktion in Gegenwart von 20 bis 100 mol-%, bezogen auf das Glyoxalmonoacetal, eines Katalysators aus einem sekundären Amin und einer Säure durchgeführt. Eine so große Katalysatormenge erfordert aus wirtschaftlichen Gründen eine Katalysatorabtrennung und - rückführung nach der Reaktion.

Es bestand daher die Aufgabe, ein technisch einfaches Verfahren zur Herstellung von But-2-en-1,4-dial-4-acetalen aus Glyoxal zu finden, das die oben beschriebenen Nachteile bekannter Verfahrensschritte vermeidet.

Demgemäß wurde das eingangs definierte Verfahren gefunden, das dadurch gekennzeichnet ist, daß man
a) Glyoxal mit einem 1,3-Propandiol der allgemeinen Formel II 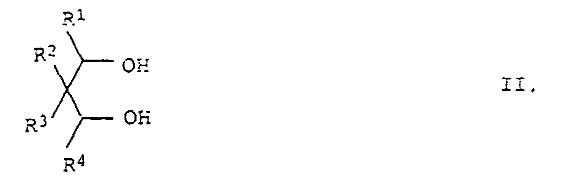 in wäßriger Lösung in Gegenwart einer Säure zu einem Monoacetal der allgemeinen Formel III 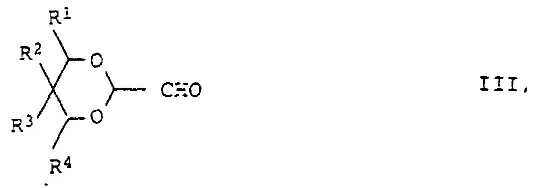 umsetzt, neutralisiert und gegebenenfalls die leichter als das Monoacetal III flüchtigen Bestandteile abdestilliert,
b) das so erhaltene, das Monoacetal der Formel III enthaltende Reaktionsgemisch mit einem Aldehyd R⁵-CH₂-CHO in Gegenwart von 0,01 bis 10 mol-%, bezogen auf Glyoxal, eines Katalysatorgemisches aus einem sekundären Amin und einer Säure zu einem Aldol der allgemeinen Formel IV 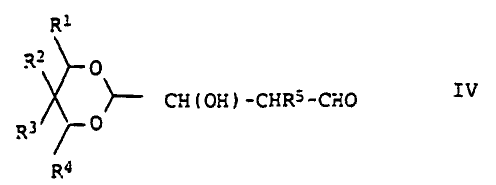 umsetzt, gegebenenfalls die leichter als das Aldol IV flüchtigen Bestandteile abdestilliert, und
c) das Aldol IV in Gegenwart von Acetanhydrid zum Produkt I dehydratisiert.

Weiterhin wurden neue Halbacetale von Glyoxalmonoacetalen gefunden.

### Verfahrensschritt a)

Glyoxal wird vorzugsweise als wäßrige Lösung eingesetzt, wobei man zweckmäßigerweise die üblichen technischen wäßrigen Lösungen mit einem Glyoxalgehalt von 20 bis 60, vorzugsweise 30 bis 50 Gew.-% verwendet.

Die 1,3-Propandiole der allgemeinen Formel II tragen als Reste R¹ bis R⁴ Wasserstoffatome oder aliphatische Reste mit 1 bis 6 Kohlenstoffatomen. Diese Reste können geradkettige, verzweigte oder cycloaliphatische Reste sein, wie Alkylgruppen mit vorzugsweise 1 bis 4 Kohlenstoffatomen oder Cycloalkylgruppen mit 4 bis 6 Kohlenstoffatomen. Die Reste R¹ und R² oder R² und R³ können auch jeweils gemeinsame Glieder eines aliphatischen 4- bis 7-gliedrigen. vorzugsweise 5- bis 6-gliedrigen Ringes sein, der ein Heteroatom, je ein Sauerstoff- oder Stickstoffatom enthalten kann. Beispielsweise seien die folgenden aliphatischen Reste genannt: Methyl, Ethyl, Propyl, Isopropyl, Butyl und Cyclohexyl. Als aromatische Reste kommen z.B. Phenylreste in Betracht. Aliphatische 4- bis 7-gliedrige Ringe, die noch ein Heteroatom enthalten können, sind z.B. Cyclopentan, Cyclohexan oder Tetrahydropyran. Im einzelnen seien die folgenden 1,3-Propandiole genannt: 2,2-Dimethylpropandiol-(1,3), 2-Ethyl-2-methylpropandiol-(1,3), 2-Butyl-2-ethylpropandiol-(1,3), 1-Isopropyl-(2,2-dimethylpropandiol-(1,3) und 1-Propyl-2-ethylpropandiol-(1,3).

Pro Mol Glyoxal werden in der Regel 0,1 bis 5 Mol, vorzugsweise 0,5 bis 2 Mol des 1,3-Propandiols der Formel II eingesetzt.

Die Umsetzung des Glyoxals mit dem 1,3-Propandiol wird in Gegenwart katalytischer Mengen Säure vorgenommen. Als Säuren kommen hierfür z.B. Mineralsäuren wie Schwefelsäure, Sulfonsäuren wie Methansulfonsäure, p-Toluolsulfonsäure, Carbonsäuren wie Trichloressigsäure und Oxalsäure, aber auch saure Ionenaustauscher in Betracht. Im allgemeinen verwendet man die Säuren in Mengen von 0,01 bis 0,25 Mol pro Mol Glyoxal.

In einer bevorzugten Ausführungsform des erfindungsgemäßen Verfahrens nimmt man die Umsetzung in Gegenwart eines Lösungsmittels vor, welches mit dem wäßrigen Ausgangsgemisch zwei Phasen bildet. Als Lösungsmittel kommen z.B. aliphatische, cycloaliphatische oder aromatische Kohlenwasserstoffe, die z.B. Halogenatome, Hydroxylgruppen und/oder Alkoxygruppen enthalten können und zwei bis 15 Kohlenstoffatome aufweisen, in Betracht. Das sind z.B. aliphatische und cycloaliphatische Kohlenwasserstoffe wie n-Hexan, i-Hexan, isomere Heptane, Octane, Cyclohexan, Halogenalkan, wie 1,2-Dichlorethan, 1,2-Dichlorpropan, Trichlormethan, Tetrachlormethan, 1,1,1- und 1,1,2-Trichlorethan, 1,1,2-Trichlorethen, Alkohole wie Butanole, Pentanole, Hexanole, Ethylhexanol, Nonanole, Decanole und Undecanole, oder ggf. substituierte aromatische Kohlenwasserstoffe, wie Benzol, Toluol, Xylol, Ethylbenzol, Isopropylbenzol, Durol, Tetralin, Chlorbenzol, 1,2-Dichlorbenzol oder Anisol. Diese Lösungsmittel, die auch im Gemisch eingesezt werden können, werden z.B. in Mengen von 100 bis 200 ml, vorzugsweise 150 bis 1000 ml pro Mol eingesetzten Diols zugegeben. Besonders vorteilhafte Ergebnisse werden erhalten, wenn man als Lösungsmittel Gemische aus Alkanolen wie Butanol, Pentanol oder Hexanol einerseits und aromatischen Kohlenwasserstoffen wie Benzol, Toluol oder Xylol andererseits verwendet. Die Reaktion kann bei Normaldruck, ebenso aber auch bei vermindertem oder erhöhtem Druck durchgeführt werden. Vorzugsweise beträgt der Reaktionsdruck 1 bis 5 bar.

Die Reaktionstemperatur beträgt im allgemeinen 20 bis 150°C. Das Verfahren kann sowohl diskontinuierlich wie auch kontinuierlich durchgeführt werden. Die Verweilzeit beträgt dabei im allgemeinen 1 bis 10 Stunden.

Nach Beendigung der Reaktion wird die Reaktionslösung durch Zugabe einer Base wie z.B. Natriumcarbonat, Natriumhydrogencarbonat, Natriumhydroxid neutralisiert. Auch Ammoniak, primäre Amine, sekundäre Amine wie Dimethylamin und Diethylamin sowie tertiäre Amine wie Triethylamin kommen als Neutralisationsmittel in Betracht.

Wird die Reaktion in einem zweiphasigen Lösungsmittelgemisch durchgeführt, kann die produkthaltige organische Phase abgetrennt werden und ebenfalls neutralisiert werden. Zweckmäßigerweise wird die wäßrige Phase mehrmals mit einem der oben genannten Lösungsmittel, z.B. Butanol, extrahiert. Diese Extrakte werden dann mit der organischen Phase vereint.

Vor ihrer weiteren Umsetzung können die so erhaltenen Lösungen eingeengt werden, indem die leichter als das Monoacetal III flüchtigen Bestandteile abdestilliert werden. Besonders bevorzugt ist es, daß ein entweder bereits als Lösungsmittel eingesetzter Monoalkohol in der Lösung verbleibt oder daß ein solcher Monoalkohol zugesetzt wird. Dadurch wird erreicht, daß das Glyoxalmonoacetal in ein Halbacetal überführt wird und so vor Oligomerisierung geschützt wird.

### Verfahrensschritt b)

Die nach Verfahrensschritt (a) hergestellten Monoacetale III werden mit einem Aldehyd R⁵CH₂-CHO umgesetzt. Der Rest R⁵ steht dabei für einen Alkyl-, Alkenyl- oder Alkinylrest mit 1 bis 12 C-Atomen, der durch cycloaliphatische, aromatische oder heterocyclische Reste oder durch Hydroxy-, Ether-, Thioether-, Acyl-, Alkylamino-, Carboxi- oder Carbalkoxygruppen substituiert sein kann, einen gegebenenfalls substituierten Arylrest oder eine Alkoxi-, Alkylthio- oder Acyloxygruppe bedeutet. Vorzugsweise ist R⁵ Ethyl, Propyl und Butyl. Als Aldehyde kommen dementsprechend Verbindungen wie Propanal, Butanal, Pentanal, Isopentanal, Hexanal, Isohexanal, Decanal und Undecanal in Betracht.

Als Katalysatorsystem wird eine Mischung aus einem sekundären Amin und einer Säure verwendet. Als Säuren kommen bevorzugt Carbonsäuren mit 1 bis 10 Kohlenstoffatomen in Betracht, z.B. Essigsäure, Propionsäure, Butansäure, Pentansäure, Hexansäure und 2-Ethylhexansäure. Ebenso können Dicarbonsäuren wie Oxalsäure oder Bernsteinsäure verwendet werden. Als Aminkomponente sind Dialkylamine wie Dimethylamin, Diethylamin, Diisopropylamin, Din-Butylamin, Methylethylamin, Methylbutylamin zu nennen, weiterhin Hydroxyalkylamine wie Diethanolamin, Methylethanolamin oder cyclische Amine, die noch weitere Heteroatome tragen können wie Pyrrolidin, Piperidin und Morpholin. Die Katalysatorsysteme verwendet man in Mengen von 0,01 bis 10 mol-%, bezogen auf Glyoxal, bevorzugt sind 0,1 bis 8 mol-%. Das Verhältnis von Amin zu Säure muß nicht exakt äquimolar sein. Vielmehr kann es von Vorteil sein, mit einem geringen Säureüberschuß zu arbeiten. Es hat sich als zweckmäßig erwiesen, das Monoacetal III aus Verfahrensschritt (a) vorzulegen und das Katalysatorsystem zuzugeben. Die Reaktionstemperatur bei dieser Aldolreaktion beträgt im allgemeinen 20 bis 90°. vorzugsweise 30 bis 80°C.

Die Reaktionszeit beträgt in der Regel 15 bis 240 Minuten.

Die so erhaltenen Reaktionslösungen werden vor einer weiteren Umsetzung vorzugsweise eingeengt, indem die leichter als das Aldol IV flüchtigen Bestandteile abdestilliert werden.

### Verfahrensschritt c)

Zur Herstellung der Verfahrensprodukte I können die Aldole IV, vorzugsweise ohne Zwischenisolierung, in an sich bekannter Weise mit Acetanhydrid umgesetzt werden.

Man geht vorzugsweise so vor, daß man zu dem erfindungsgemäß erhaltenen Reaktionsgemisch Acetanhydrid in Mengen von 1 bis 5 Mol pro Mol Aldol gibt sowie ggf. geringe Mengen eines Katalysators wie Natriumacetat oder 4-Dimethylamino-pyridin zufügt, und anschließend noch 1 bis 12, vorzugsweise 2 bis 6 Stunden unter Rückfluß zum Sieden erhitzt.

Die Verfahrensprodukte I können aus den Reaktionslösungen nach an sich bekannten Methoden, vorzugsweise destillativ isoliert werden.

Das erfindungsgemäße Verfahren erlaubt eine einfache technische Herstellung der Verfahrensprodukte I aus Glyoxal. Bemerkenswerterweise können die im Verfahrensschritt (a) hergestellten Monoacetale ohne destillative Isolierung mit einem Aldehyd zu den Aldolen IV umgesetzt werden, ohne daß die in der Lösung erhaltenen Nebenprodukte stören. Weiterhin ist es möglich, im Verfahrensschritt (b) so geringe Katalysatormengen einzusetzen, daß eine Kacalysatoraufarbeitung und -rückführung nicht notwendig ist.

Die Verfahrensprodukte sind wertvolle Vorprodukte für die Herstellung von Terpenen und terpenanaloger Verbindungen mit biologischer und pharmakologischer Wirksamkeit.

Die Erfindung betrifft auch neue Halbacetale von Glyoxalmonoacetalen V 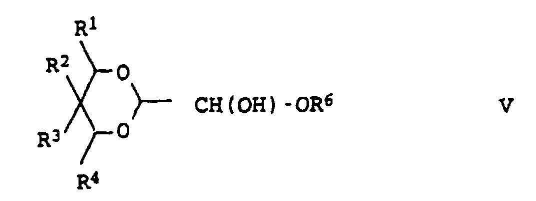 in der R⁶ für einen geradkettigen oder verzweigten C₁-C₁₂-Alkylrest, einen C₆-C₁₂-Arylrest oder einen C₇-C₂₀-Aralkylrest steht.

Im einzelnen steht der Rest R⁶ bevorzugt für Alkylreste wie Methyl, Ethyl, Propyl, Butyl, für Arylreste wie Phenyl oder Aralkylreste wie Benzyl.

Diese Produkte V können erhalten werden, indem man den Reaktionsaustrag aus Verfahrensschritt (a) nach Zugabe eines Monoalkohols R⁶-OH destillativ aufarbeitet. Die Halbacetale V können in für die freien Aldehyde typischen Reaktionen, beispielsweise Verfahrensschritt (b), anstelle der Aldehyde eingesetzt werden. Sie haben gegenüber den Aldehyden den Vorteil, daß sie problemlos gelagert werden können, ohne daß sie oligomerisieren.

### Beispiele

### 1. Herstellung von 2-Methylbut-2-en-1,4-diol-4-neopentyl-glykolacetal

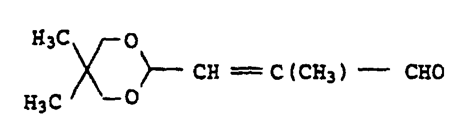

### Verfahrensstufe a)

Eine Mischung aus 234 kg (2250 Mol) 2,2-Dimethylpropan-1,3-diol, 326 kg (2250 Mol) 40 %igem Glyoxal in Wasser, 167 kg 1-Butanol und 900 1 Toluol wurden mit 22,5 kg 50 %iger Schwefelsäure versetzt und bei einem Eigendruck von 2,5 bis 3 bar 2 Stunden auf 140°C erhitzt. Nach Abkühlen auf Raumtemperatur wurde die wäßrige Phase abgetrennt und mehrfach mit 1-Butanol extrahiert. Die Butanolextrakte wurden mit der organischen Phase vereint, mit einer Lösung von 5 kg Natriumcarbonat in 30 kg Wasser neutralisiert und von der wäßrigen Phase getrennt. Aus der organischen Phase wurde Toluol vollständig und 1-Butanol zu ca. 50 % im Vakuum abdestilliert.

### Verfahrensstufe b)

Der Rückstand aus Verfahrensstufe a) wurde mit 175 kg (3020 Mol) Propionaldehyd versetzt und bei 60 bis 80°C mit 17,5 kg Katalysatorlösung, bestehend aus 3,1 kg (51 mol) Essigsäure, 5,70 kg (51 Mol) 40 %igem Dimethylamin und 8,7 kg Wasser, versetzt. Nach einer Reaktionszeit von 30 min bei 85°C wurden im Vakuum alle Bestandteile, die flüchtiger als das Aldolprodukt waren, abdestilliert.

### Verfahrensstufe c)

Der Rückstand aus Verfahrensstufe b) wurde bei 110°C mit 275 kg (2700 Mol) Essigsäureanhydrid versetzt und 3 h auf 140°C erhitzt. Nach Destillation wurden 227,7 kg 2-Methylbut-2-en-1,4-dial-4-neopentylglykolacetal isoliert, was einer Ausbeute von 55 %, bezogen auf Glyoxal, entspricht.

### 2. Herstellung von 2-(1-Hydroxy-2-oxa-hexyl)-5,5-dimethyl-1,3-dioxan

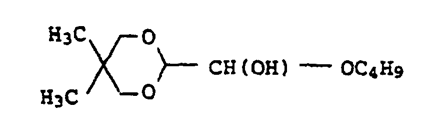

4800 g des Destillationsrückstandes der Verfahrensstufe (a) aus Beispiel 1 wurden über eine Füllkörperkolonne destilliert. Bei einem Druck von 1 - 2 mbar wurden bei 46 - 50°C 3400 g 2-(1-Hydroxy-2-oxa-hexyl)-5,5-dimethyl-1,3-dioxan in Form eines farblosen Öls isoliert. Die Ausbeute betrug 70 %, bezogen auf den Rückstand aus Verfahrensstufe (a).

¹H-NMR(CDCl₃) : 0.73 (s, CH₃), 0.90 (m, CH₃), 1.20 (s, CH₃), 1.38 (m, CH₂), 1.60 (m, CH₂), 3.50 (m, CH), 3.55 (m, CH₂), 3.65 (m, CH₂), 3.85 (CH), 4.60 ppm (br s, OH).

¹³C-NMR (CDCl₃) : 13.9 (1C, CH₃), 19.2 (1C, CH₂), 21.7 (1C, CH₃), 22.9 (1C, CH₃), 30.4 (1C), 34.8 (1C, CH₂), 67.9 (1C, CH₂), 76.8 (2C, CH₂), 95.7 (1C, CH), 100.2 ppm (1C, CH).

## Patentansprüche

1. Verfahren zur Herstellung von 2-substituierten But-2-en-1,4-dial-4-acetalen der allgemeinen Formel I 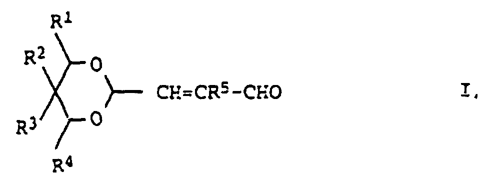 in der die Substituenten R¹ bis R⁴ für Wasserstoff oder C₁-C₆-aliphatische Reste stehen und R² und R³ oder R¹ und R² jeweils gemeinsame Glieder eines aliphatischen 4- bis 7-gliedrigen Ringes, der ein Heteroatom enthalten kann, sind, und in der R⁵ einen Alkyl-, Alkenyl- oder Alkinylrest mit 1 bis 12 C-Atomen, der durch cycloaliphatische, aromatische oder heterocyclische Reste oder durch Hydroxy-, Ether-, Thioether-, Acyl-. Alkylamino-, Carboxi- oder Carbalkoxygruppen substituiert sein kann, einen gegebenenfalls substituierten Arylrest oder eine Alkoxi-, Alkylthio- oder Acyloxygruppe bedeutet, **dadurch gekennzeichnet, daß** man
a) Glyoxal mit einem 1,3-Propandiol der allgemeinen Formel II 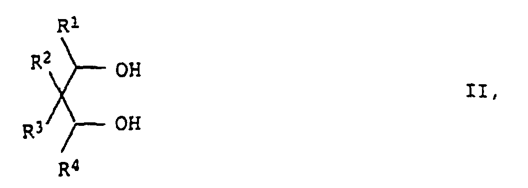 in wäßriger Lösung in Gegenwart einer Säure zu einem Monoacetal der allgemeinen Formel III 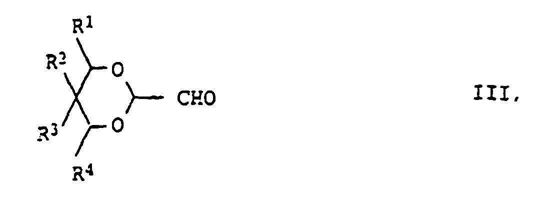 umsetzt, die Reaktionslösung neutralisiert und gegebenenfalls die leichter als das Monoacetal III flüchtigen Bestandteile abdestilliert,
b) das so erhaltene, das Monoacetal der Formel III enthaltende Reaktionsgemisch mit einem Aldehyd R⁵-CH₂-CHO in Gegenwart von 0,01 bis 10 Mol-%, bezogen auf Glyoxal, eines Katalysatorgemisches aus einem sekundären Amin und einer Säure zu einem Aldol der allgemeinen Formel IV 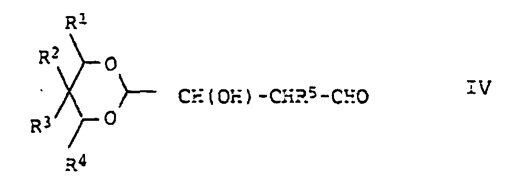 umsetzt, gegebenenfalls die leichter als das Aldol IV flüchtigen Bestandteile abdestilliert, und
c) das Aldol IV in Gegenwart von Acetanhydrid zum Produkt I dehydratisiert.

2. Verfahren nach Anspruch 1, **dadurch gekennzeichnet, daß** man Verfahrensschritt (a) in Gegenwart eines Lösungsmittels vornimmt, das mit dem wäßrigen Ausgangsgemisch zwei Phasen bildet.

3. Verfahren nach Anspruch 2, **dadurch gekennzeichnet, daß** man als Lösungsmittel ein Gemisch aus Butanol, Pentanol oder Hexanol einerseits und Benzol, Toluol oder Xylol andererseits verwendet.

4. Verfahren nach Anspruch 1, **dadurch gekennzeichnet, daß** man als Aldehyd im Verfahrensschritt (b) Propionaldehyd verwendet.

5. Verfahren nach Anspruch 1, **dadurch gekennzeichnet, daß** man 2-Methylbut-2-en-1,4-dial-4-neopentylglykolacetal herstellt.

6. Verfahren nach Anspruch 1, **dadurch gekennzeichnet, daß** man im Verfahrensschritt c) zu dem im Verfahrensschritt b) erhaltenen Reaktionsgemisch 1 bis 5 Mol Acetanhydrid pro Mol Aldol, sowie ggf. geringe Mengen von Natriumacetat oder 4-Dimethylamino-pyridin als Katalysator zufügt, und anschließend noch 1 bis 12 Stunden unter Rückfluß zum Sieden erhitzt.

## Claims

1. A process for preparing 2-substituted but-2-ene-1,4-dial 4-acetals of the general formula I 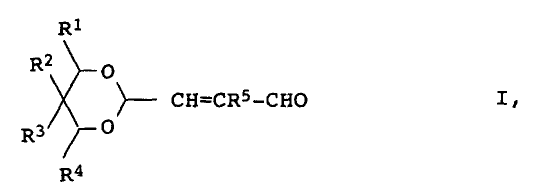 where R¹ to R⁴ are each hydrogen or an aliphatic C₁-C₆ radical and R² and R³ or R¹ and R² are pairs of common members of an aliphatic ring which has from 4 to 7 members and may contain a hetero atom, and where R⁵ is an alkyl, alkenyl or alkynyl radical having from 1 to 12 carbon atoms with or without substitution by cycloaliphatic, aromatic or heterocyclic radicals or by hydroxyl, ether, thioether, acyl, alkylamino, carboxyl or carbalkoxy groups or is a substituted or unsubstituted aryl radical or an alkoxy, alkylthio or acyloxy group, which comprises
a) reacting glyoxal with a 1,3-propanediol of the general formula II 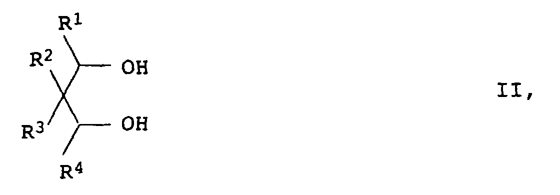 in aqueous solution in the presence of an acid to form a monoacetal of the general formula III 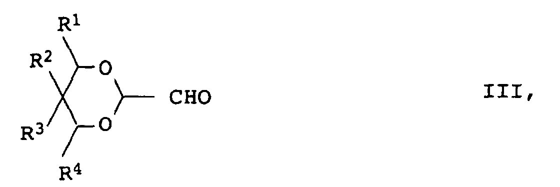 neutralizing the reaction solution and optionally distilling the neutralized reaction solution to remove constituents which are more volatile than said monoacetal III,
b) reacting the resulting reaction mixture, comprising the monoacetal of the formula III, with an aldehyde R⁵-CH₂-CHO in the presence of from 0.01 to 10 mol%, based on glyoxal, of a catalyst mixture of a secondary amine and an acid to form an aldol of the general formula IV 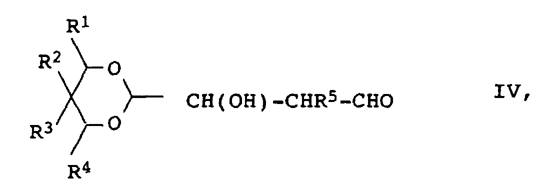 optionally distilling to remove constituents which are more volatile than said aldol IV, and
c) dehydrating said aldol IV in the presence of acetic anhydride to obtain product I.

2. A process as claimed in claim 1, wherein step (a) is carried out in the presence of a solvent which forms two phases with the aqueous starting mixture.

3. A process as claimed in claim 2, wherein the solvent used is a mixture of butanol, pentanol or hexanol on the one hand and benzene, toluene or xylene on the other.

4. A process as claimed in claim 1, wherein the aldehyde used in step (b) is propionaldehyde.

5. A process as claimed in claim 1 for preparing 2-methylbut-2-ene-1,4-dial 4-(2,2-dimethyl-3-hydroxypropyl) acetal.

6. A process as claimed in claim 1, wherein, in step c), the reaction mixture obtained in step b) is admixed with from 1 to 5 mol of acetic anhydride per mole of aldol and also, if necessary, small amounts of sodium acetate or 4-dimethylaminopyridine as catalyst and then refluxed for 1-12 hours.

## Revendications

1. Procédé pour la préparation de but-2-ène-1,4-dial-4-acétals 2-substitués de formule générale I 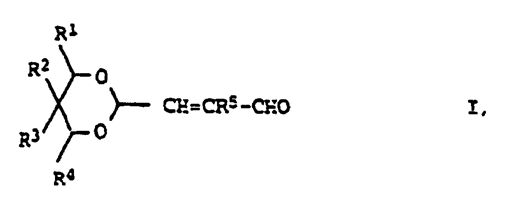 dans laquelle les substituants R¹ à R⁴ désignent l'hydrogène ou des restes aliphatiques en C₁-C₆, et R² et R³ ou R¹ et R² sont à chaque fois des membres communs d'un cycle aliphatique ayant 4 à 7 chaînons, qui peut contenir un hétéroatome, et où R⁵ désigne un reste alkyle, alcényle ou alcynyle ayant 1 à 12 atomes de carbone, qui peut être substitué par des restes cycloaliphatiques, aromatiques ou hétérocycliques ou par des groupes hydroxy, éther, thioéther, acyle, alkylamino, carboxy ou carbalcoxy, un reste aryle ébentuellement substitué ou un groupe alcoxy, alkylthio ou acyloxy, **caractérisé par le fait que**
a) on fait réagir du glyoxal avec un 1,3-propanediol de formule générale II 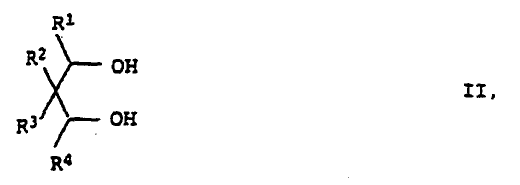 en solution aqueuse en présence d'un acide pour former un monoacétal de formule générale III 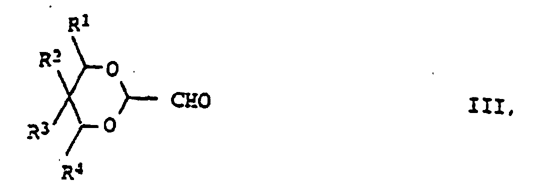 on neutralise la solution de réaction et on sépare éventuellement par distillation les constituants plus volatils que le monoacétal III,
b) on fait réagir le mélange réactionnel ainsi obtenu, contenant le monoacétal de formule III, avec un aldéhyde R⁵-CH₂-CHO en présence de 0,01 à 10 % en moles, par rapport au glyoxal, d'un mélange catalyseur composé d'une amine secondaire et d'un acide pour former un aldol de formule générale IV 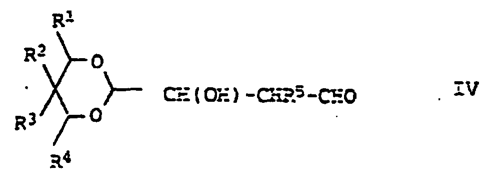 éventuellement on sépare par distillation les constituants plus volatils que l'aldol IV, et
c) on déshydrate l'aldol IV en présence d'anhydride acétique pour former le produit I.

2. Procédé selon la revendication 1, **caractérisé par le fait qu'**on conduit l'étape opératoire (a) en présence d'un solvant qui forme deux phases avec le mélange aqueux de départ.

3. Procédé selon la revendication 2, **caractérisé par le fait qu'**on utilise comme solvant un mélange de butanol, pentanol ou hexanol d'une part et de benzène, toluène ou xylène d'autre part.

4. Procédé selon la revendication 1, **caractérisé par le fait qu'**on utilise l'aldéhyde propionique comme aldéhyde dans l'étape opératoire (b).

5. Procédé selon la revendication 1, **caractérisé par le fait qu'**on prépare le 2-méthylbut-2-ène-2,4-dial-4-néopentylglycolacétal.

6. Procédé selon la revendication 1, **caractérisé par le fait qu'**on ajoute dans l'étape opératoire (c), au mélange réactionnel obtenu dans l'étape opératoire (b), 1 à 5 moles d'anhydride acétique par mole d'aldol, ainsi qu'éventuellement de faibles quantités d'acétate de sodium ou de 4-diméthylamino-pyridine, à titre de catalyseur, et on chauffe ensuite encore pendant 1 à 12 heures à ébullition sous reflux.
